# EUROPÄISCHE PATENTSCHRIFT

(11) **EP 1 335 943 B1**
(45) Veröffentlichungstag und Bekanntmachung des Hinweises auf die Patenterteilung: **20.07.2005**
(21) Anmeldenummer: 01993637.6
(22) Anmeldetag: 09.11.2001
(51) Int. Cl.: C08F 220/18, A61K 7/06

(54) **ACRYLATPOLYMERISATE AUF BASIS VON TERT.BUTYLACRYLAT UND/ODER TERT.-BUTYLMETHACRYLAT**
ACRYLATE POLYMERS BASED ON TERT-BUTYL ACRYLATE AND/OR TERT-BUTYL METHACRYLATE
POLYMERISATS D'ACRYLATE A BASE DE BUTYLACRYLATE TERT. ET/OU DE BUTYLMETHACRYLATE TERT.

(30) Priorität: 10.11.2000 DE 10055776
(43) Veröffentlichungstag der Anmeldung: 20.08.2003
(73) Patentinhaber: BASF AKTIENGESELLSCHAFT, 67056 Ludwigshafen (DE)
(72) Erfinder: DAUSCH, Wilma, M., 67117 Limburgerhof (DE); ZEITZ, Katrin, 68239 Mannheim (DE); SCHNEIDER, Tanja, 64625 Bensheim (DE); ANGEL, Maximilian, 67105 Schifferstadt (DE); DE POTZOLLI, Bernd, 67098 Bad Dürkheim (DE); WOOD, Claudia, 69469 Weinheim (DE)
(86) Internationale Anmeldenummer: PCT/EP2001/012976
(87) Internationale Veröffentlichungsnummer: WO 2002/038638

(56) Entgegenhaltungen:
- EP-A- 0 379 082
- WO-A-00/64983
- DE-A- 19 908 183

## Beschreibung

Die vorliegende Erfindung betrifft neue Polymerisate auf Basis von tert.-Butylacrylat und/oder tert.-Butylmethacrylat, Verfahren zu ihrer Herstellung sowie ihre Verwendung, insbesondere in kosmetischen Zubereitungen.

Aus der EP 379 082 A1 sind Copolymerisate auf der Basis von tert.-Butylacrylat und/oder tert.-Butylmethacrylat mit einem K-Wert von 10 bis 50 bekannt, die durch radikalische Polymerisation von
A) 75 bis 99 Gew.-% tert.-Butylacrylat und/oder tert.-Butylmethacrylat,
B) 1 bis 25 Gew.-% Acrylsäure und/oder Methacrylsäure und
C) 0 bis 10 Gew.-% eines weiteren radikalisch copolymerisierbaren Monomeren
erhältlich sind, wobei die Carboxylgruppen der Copolymerisate nicht, teilweise oder vollständig durch Amine neutralisiert sind.

Aus der EP 696 916 B1 sind Copolymerisate auf Basis von tert.-Butylacrylat oder tert.-Butylmethacrylat mit einem K-Wert von 10 bis 50 bekannt, die durch radikalische Polymerisation von
A) 30 bis 72 Gew.-% tert.-Butylacrylat oder tert.-Butylmethacrylat oder einer Mischung hieraus als Monomerem A,
B) 10 bis 28 Gew.-% Acrylsäure oder Methacrylsäure oder einer Mischung hieraus als Monomerem B und
C) 0 bis 60 Gew.-% eines radikalisch copolymerisierbaren Monomeren oder einer radikalisch copolymerisierbaren Monomerenmischung als Monomeren C, wobei mindestens eines der Monomeren C ein Homopolymerisat mit einer Glastemperatur kleiner als 30°C liefert,
erhältlich sind, wobei die Carboxylgruppen der Copolymerisate nicht, teilweise oder vollständig neutralisiert sind.

Die beschriebenen Polymerisate eignen sich als Filmbildner in kosmetischen Mitteln. Nachteilig an den bekannten Polymerisaten ist vor allen ihr starker Eigengeruch, der z.T. bei der Lagerung und/oder Formulierung in kosmetischen Mitteln noch zunimmt. Dies führt dazu, daß die bekannten Produkte nur begrenzt einsetzbar sind. In kosmetischen Formulierungen wird versucht, diesen Eigengeruch der Polymerisate durch den Einsatz von Parfümölen zu überdecken. Abgesehen davon, daß die vollständige Überdeckung des Eigengeruchs nicht immer möglich ist, führt der Einsatz von Parfümölen in Einzelfällen zu unerwünschten allergischen Reaktionen. Dies limitiert den Einsatz der bekannten Polymerisate in kosmetischen Mitteln. Darüber hinaus sollten die Polymerisate an sich gegenüber den Produkten des Standes der Technik reizärmer sein und somit für den Einsatz in anti-allergenen kosmetischen Zubereitungen geeignet sein.

Die Aufgabe der vorliegenden Erfindung hat somit darin bestanden verbesserte Polymerisate auf Basis von tert.-Butylacrylat und/oder tert.-Butylmethacrylat zur Verfügung zu stellen, die aufgrund ihres neutralen Geruchs für ein weites Einsatzspektrum, insbesondere in kosmetischen Mitteln geeignet sind und sich insbesondere auch für Formulierungen ohne den Zusatz von Parfümölen eignen. Hierbei ist insbesondere von Interesse, daß die Polymerisate in kosmetischen Zubereitungen auch nach Lagerung keinen Eigengeruch entwickeln. Daneben sind anwendungstechnischen Eigenschaften, wie Auswaschbarkeit aus dem Haar, Verträglichkeit mit weiteren kosmetischen Inhaltsstoffen, insbesondere Löslichkeit in wasserhaltigen Zubereitungen, Griff und Festigung des behandelten Haars aufweisen erwünscht. Auch die Bereitstellung von Polymerisaten, die gegenüber den Produkten des Standes der Technik reizärmer sind, ist wünschenswert.

Die Aufgabe wird gelöst durch Acrylatpolymerisate mit einem K-Wert von 10 bis 60, erhältlich durch radikalische Polymerisation von
A) 30 bis 99 Gew.-% tert.-Butylacrylat und/oder tert.-Butylmethacrylat als Monomerem A,
B) 1 bis 28 Gew.-% Acrylsäure und/oder Methacrylsäure als Monomerem B und
C) 0 bis 60 Gew.-% eines radikalisch copolymerisierbaren Monomeren oder einer radikalisch copolymerisierbaren Monomerenmischung als Monomerem C, wobei mindestens eines der Monomeren C ein Homopolymerisat mit einer Glastemperatur kleiner als 30°C liefert,
mit der Maßgabe, daß sich die Gew.% zu 100 addieren,
in Gegenwart von Alkanthiolen mit der C-Kettenlänge von C 14 bis C 22.

Die Aufgabe wird gelöst durch Acrylatpolymerisate mit einem K-Wert von 10 bis 60, erhältlich durch radikalische Polymerisation von
A) 30 bis 99 Gew.-% tert.-Butylacrylat und/oder tert.-Butylmethacrylat als Monomerem A,
B) 1 bis 28 Gew.-% Acrylsäure und/oder Methacrylsäure als Monomerem B und
C) 0 bis 60 Gew.-% eines radikalisch copolymerisierbaren Monomeren oder einer radikalisch copolymerisierbaren Monomerenmischung als Monomerem C, wobei mindestens eines der Monomeren C ein Homopolymerisat mit einer Glastemperatur kleiner als 30°C liefert,
mit der Maßgabe, daß sich die Gew.% zu 100 addieren,
in Gegenwart von Alkanthiole mit der C-Kettenlänge von C 10 bis C 22
und anschließender Wasserstoffperoxid-Behandlung,

Im Gegensatz zu den Produkten des Standes der Technik, insbesondere zu Polymerisaten gemäß EP 696 916, zeichnen sich die so erhältlichen Polymerisate durch Geruchsfreiheit aus und entwickeln auch bei Lagerung sowohl als Einzelsubstanzen als auch in kosmetischen Zubereitungen keinen Geruch. Gleichzeitig zeigen die so erhältlichen Polymerisate gute filmbildende Eigenschaften und gute Verträglichkeit mit üblichen kosmetischen Inhaltsstoffen.

Die Acryatpolymerisate werden in bekannter Weise durch radikalische Polymerisation der Monomeren A, B und gegebenenfalls C hergestellt. Hierbei arbeitet man nach den üblichen Polymerisationstechniken, zum Beispiel nach den Methoden der Suspensions-, Emulsions- oder Lösungspolymerisation.

Die Herstellung der Polymerisate erfolgt in üblicher Weise unter Verwendung von Initiatoren, wie Peroxo- oder Azoverbindungen, beispielsweise Dibenzoyloxid, t-Butylperpivalat, t-Butyl-per-2-ethylhexanoat, Di-t-Butylperoxid, t-Butylhydroperoxid, 2,5-Dimethyl-2,5-di(t)butylperoxy(hexan), Alkalimetall- oder Ammoniumpersulfate, Azo-bis-isobutyronitril, 2,2'-Azo-bis-(2-Methylbutyronitril), 2,2'-Azo-bis-(2,4-dimethylvaleronitril), 1,1'-Azo-bis-(1-cyclohexancarbonitril), 2,2'-Azobis(2-amidinopropan)salze, 4,4'-Azobis(4-Cyanovaleriansäure) oder 2-(Carbamoylazo)-isobutyronitril etc., Wasserstoffperoxid oder Redoxinitiatoren. Die Initiatoren werden üblicherweise in Mengen bis zu 10, vorzugsweise 0,02 bis 5 Gew.-%, bezogen auf die zu polymerisierenden Monomeren eingesetzt.

Die Emulsionspolymerisation erfolgt üblicherweise unter Sauerstoffausschluß bei Temperaturen im Bereich von 20 bis 200°C. Die Polymerisation kann diskontinuierlich oder kontinuierlich durchgeführt werden.

Vorzugsweise dosiert man zumindest einen Teil der Monomere, Initiatoren und Alkanthiole während der Polymerisation gleichmäßig in das Reaktionsgefäß. Die Monomere, das Alkanthiol und der Initiator können jedoch auch im Reaktor vorgelegt und polymerisiert werden, wobei gegebenenfalls gekühlt werden muß.

Nach beendeter Polymerisation kann man zur Senkung des Restmonomerengehaltes eine Nachpolymerisation unter Zugabe geeigneter Initiatoren in bekannter Weise durchführen. Gewünschtenfalls kann auch eine physikalische Desodorierung in üblicher Weise erfolgen, beispielsweise durch Einleiten von Wasserdampf.

Als Alkanthiole werden lineare und verzweigte Alkanthiole mit einer C-Kettenlänge von C 10 bis C 22 eingesetzt. Besonders bevorzugt sind lineare Alkanthiole, weiterhin bevorzugt sind Alkanthiole mit einer Kettenlänge von C 14 bis C 22, insbesondere von C 14 bis C 18. Als Alkanthiole seien genannt n-Decanthiol, n-Dodecanthiol, tert.-Dodecanthiol, n-Tetradecanthiol, n-Pentadecanthiol, n-Hexadecanthiol, n-Heptadecanthiol, n-Octadecanthiol, n-Nonadecanthiol, n-Eicosanthiol, n-Docosanthiol. Besonders bevorzugt sind lineare, geradzahlige Alkanthiole.

Die Alkanthiole können auch in Mischungen eingesetzt werden.

Die Alkanthiole werden üblicherweise in Mengen von 0,1 bis 5 Gew.-%, insbesondere 0,25 bis 2 Gew.-% bezogen auf die zu polymerisierenden Monomere eingesetzt. Üblicherweise werden die Alkanthiole zusammen mit den Monomeren der Polymerisation zugesetzt.

Werden Alkanthiole mit einer C-Kettenlänge von C 10 bis C 13 eingesetzt, ist eine anschließende Wasserstoffperoxidbehandlung erforderlich, um geruchlich neutrale Polymerisate zu erhalten. Für diese sich an die Polymerisation anschließende Wasserstoffperoxidbehandlung werden üblicherweise 0,01 bis 2,0 Gew.-%, insbesondere 0,02 bis 1,0 Gew.-%, besonders bevorzugt 0,03 bis 0,15 Gew.-% Wasserstoffperoxid, insbesondere 0,1 bis 1,0 Gew.-% bezogen auf die zu polymerisierenden Monomere eingesetzt. Es hat sich als vorteilhaft erwiesen die Wasserstoffperoxidbehandlung bei einer Temperatur von 20 bis 100°C, insbesondere von 30 bis 80°C durchzuführen. Die Wasserstoffperoxidbehandlung wird üblicherweise zwischen 30 min. und 240 min., insbesondere zwischen 45 und 90 min. durchgeführt durchzuführen.

Werden Alkanthiole mit einer C-Kettenlänge von C 14 bis C 22 eingesetzt, kann die Wasserstoffperoxidbehandlung entfallen. In einer weiteren Ausführungsform der Erfindung kann jedoch auch bei dem Zusatz von Alkanthiolen mit einer Kettenlänge von C 14 bis C 22 eine Wasserstoffperoxidbehandlung angeschlossen werden.

Die Polymerisate sollen K-Werte von 10 bis 60, vorzugsweise 15 bis 50 aufweisen. Der jeweils gewünschte K-Wert läßt sich in an sich bekannter Weise durch Wahl der Polymerisations-bedingungen, beispielsweise der Polymerisationstemperatur und der Initiatorkonzentration, einstellen. Gegebenenfalls, insbesondere bei Anwendung der Emulsions- und Suspensionspolymerisation, kann der Einsatz von Reglern, insbesondere von Schwefelverbindungen wie Mercaptoethanol, 2-Ethylhexylthioglykolat, Thioglykolsäure oder Dodecylmercaptan zur Reduzierung des K-Wertes angebracht sein. Die K-Werte werden nach Fikentscher, Cellulosechemie, Bd. 13, S. 58 bis 64 (1932) bei 25°C in 1 gew.-%iger ethanolischer Lösung gemessen und stellen ein Maß für das Molgewicht dar.

Derartige Polymerisate haben üblicherweise Glastemperaturen zwischen 50 und 130°C, insbesondere zwischen 60 und 100°C.

Wird das Polymerisat durch Emulsionspolymerisation hergestellt, kann die erhaltene Dispersion entweder direkt in eine wäßrige, wäßrig-alkoholische oder alkoholische kosmetische Zubereitung, beispielsweise eine Haarfestigungszubereitung eingearbeitet werden oder es erfolgt eine Trocknung, z.B. Sprühtrocknung, der Dispersion, so daß das Polymerisat als Pulver verwendet und verarbeitet werden kann.

Das so erhaltene Polymerisat kann direkt (nicht neutralisiert) oder teilweise oder vollständig neutralisiert werden. In einer bevorzugten Ausführungsform werden die Polymerisat teilweise oder vollständig neutralisiert.

Die Neutralisation der Polymerisate erfolgt üblicherweise mit einem Alkalimetallhydroxid oder vorzugsweise mit einem Amin teilweise oder vollständig, zweckmäßigerweise zu 5 bis 100 %, vorzugsweise zu 30 bis 95 %. Die Neutralisation erfolgt bevorzugt mit
- einem Mono-, Di- oder Trialkanolamin mit 2 bis 5 C-Atomen im Alkanolrest, der gegebenenfalls in veretherter Form vorliegt, beispielsweise Mono-, Di- und Triethanolamin, Mono-, Di- und Tri-n-propanolamin, Mono-, Di- und Triisopropanolamin, 2-Amino-2-methylpropanol und Di(2-methoxyethyl)amin,
- einem Alkandiolamin mit 2 bis 5 C-Atomen, beispielsweise 2-Amino-2-methylpropan-1,3-diol und 2-Amino-2-ethylpropan-1,3-diol, oder
- einem primären, sekundären oder tertiären Alkylamin mit insgesamt 5 bis 10 C-Atomen, beispielsweise N,N-Diethylpropylamin oder 3-Diethylamino-1-propylamin. Besonders gute Ergebnisse erhielt man mit 2-Amino-2-methylpropanol, Triisopropanolamin und 2-Amino-2-ethylpropan-1,3-diol und 3-Diethylamino-1-propylamin.

Als Alkalimetallhydroxide eignen sich zur Neutralisation vor allem Natrium- und Kaliumhydroxid; weiterhin eignen sich zur Neutralisation wässerige Pufferlösungen, wie beispielsweise Puffer basierend auf Alkali- bzw. Ammoniumcarbonat oder -bicarbonat. Die Neutralisationsmittel werden vorzugsweise als verdünnte wässerige Lösung zur Dispersion gegeben.

Zur Modifikation der Eigenschaften des Acrylatpolymerisates kann gegebenenfalls noch wenigstens ein weiteres Monomer C einpolymerisiert sein. Dieses Monomer oder mindestens eines dieser Monomere soll dabei ein Homopolymerisat mit einer Glastemperatur kleiner 30 °C liefern. Vorzugsweise handelt es sich dabei um Monomere die ausgewählt sind aus der Gruppe bestehend aus C₁-C₁₈-Alkylacrylate, C₁-C₁₈-Alkylmethacrylate, N-C₁-C₁₈-Alkylacrylamiden und N-C₁-C₁₈-Alkylmethacrylamiden. Besonders bevorzugt sind N-C₁-C₄-Alkylacryl-amide oder -Methacrylamide oder Gemische von zwei oder mehreren dieser Monomere, besonders bevorzugt sind unverzweigte C₂- bis C₄-Alkylacrylate allein oder in Mischung mit verzweigten N-C₃- bis -C₄-Alkylacrylamiden. Als C₁-C₄-Alkylreste in den genannten (Meth)acrylaten und (Meth)acrylamiden kommen Methyl, Ethyl, n-Propyl, iso-Propyl, n-Butyl, iso-Butyl, sec-Butyl und t-Butyl in Betracht. Besonders bevorzugte Monomere C sind Ethylacrylat oder ein Gemisch aus Ethylacrylat und N-t-Butylacrylamid.

In einer bevorzugten Ausführungsform wird das Acrylatpolymerisat erhalten aus
A) 30 bis 72, insbesondere 50 bis 72 Gew.-%, insbesondere 60 bis 70 Gew.-% des Monomeren A,
B) 10 bis 28, insbesondere 12 bis 25 Gew.-%, insbesondere 15 bis 23 Gew.-% des Monomeren B und
C) 0 bis 60, insbesondere, 3 bis 38 Gew.-%, insbesondere 7 bis 25 Gew.-% des Monomeren C
mit der Maßgabe, daß sich die Gew.-% zu 100 addieren.

In einer weiteren bevorzugten Ausführungsform ist das Acrylatpolymerisat aus
A) tert.-Butylacrylat als Monomerem A,
B) Methacrylsäure als Monomerem B und
C) Ethylacrylat oder einer Mischung aus Ethylacrylat und N-tert.-Butylacrylamid als Monomerem C
aufgebaut.

Die erfindungsgemäßen Acrylatpolymerisate zeichnen sich durch hervorragende filmbildende Eigenschaften aus. Ein weiterer Gegenstand der Erfindung betrifft daher die Verwendung der Acrylatpolymerisate als Filmbildner.

Die erfindungsgemäßen Acrylatpolymerisate eignen sich weiterhin zur Verwendung in kosmetischen Zubereitungen. Hier eignen sich insbesondere teilweise oder vollständig neutralisierte Acrylatpolymerisate.

Als kosmetische Zubereitungen seien hautkosmetische Zubereitungen genannt, insbesondere solche zur Pflege und/oder Reinigung der Haut. Diese liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen vor. Weiterhin eignen sie sich für hautkosmetische Zubereitungen wie Gesichtswasser, Gesichtsmasken, Deodorantien und andere kosmetische Lotionen und für die Verwendung in der dekorativen Kosmetik, beispielsweise als Abdeckstift, Theaterfarbe, in Mascara und Lidschatten, Lippenstiften, Kajalstiften, Eyelinern, Makeup, Grundierungen, Rouges und Pudern und Augenbrauenstiften.

Außerdem können die erfindungsgemäßen Acrylatpolymerisate verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Die Acrylatpolymerisate können in kosmetischen Zubereitungen als wässerige oder wässerig-alkoholische Lösungen, O/W sowie W/O Emulsionen in Form von Shampoos, Cremes, Schäumen, Sprays (Pumpspray oder Aerosol), Gelen, Gelsprays, Lotionen oder Mousse vorliegen.

Besonders bevorzugt ist die Verwendung der Acrylatpolymerisate in haarkosmetischen Zubereitungen. Als haarkosmetische Zubereitungen seien genannt Haarkuren, Haarlotionen, Haarspülungen, Haaremulsionen, Spitzenfluids, Egalisierungsmittel für Dauerwellen, Hot-Oil-Treatment-Präparate, Conditioner, Curl relaxer, Styling wrap lotions, Festigerlotionen, Shampoos, Haarwachse, Pomaden, Haarschäume, Haarfärbemittel oder Haarsprays. Besonders bevorzugt ist die Verwendung der Acrylatpolymerisate in Frisurenfestiger, die in Form von Sprayzubereitungen und/oder Haarschäumen vorliegen.

Die erfindungsgemäßen Acrylatpolymerisate zeichnen sich in haarkosmetischen Zubereitungen durch ihre hohe Verträglichkeit mit den unpolaren Treibmitteln in Sprayzubereitungen, insbesondere mit Kohlenwasserstoffen wie n-Propan, iso-Propan, n-Butan, isoButan, n-Pentan und Mischungen daraus aus. Sie weisen eine gute haarfestigende Wirkung auf und zeichnen dadurch aus, daß sie das Haar praktisch nicht verkleben.

Neben der Geruchsfreiheit weisen die Acrylatpolymerisate bei den anwendungstechnischen Eigenschaften in haarkosmetischen Zubereitungen hervorragende Ergebnisse auf. Sie sind in Alkoholen wie Ethanol oder Isopropanol und in Gemischen dieser Alkohole mit Wasser klar löslich. Die Klarheit der Lösungen bleibt auch erhalten, wenn die Lösungen in Standard-Sprayformulierungen zusammen mit Treibmitteln wie Dimethylether eingesetzt werden. Die erfindungsgemäßen Haarfestigungsmittel sind einwandfrei aus dem Haar auswaschbar. Mit ihnen behandeltes Haar weist eine erhöhte Geschmeidigkeit und einen angenehmen natürlichen Griff auf. Die Festigungswirkung ist gleichzeitig dabei hoch, so daß prinzipiell eine Senkung der benötigten Menge an Filmbildner in der Haarsprayformulierung möglich ist. Aufgrund der Geruchsfreiheit der Acrylatpolymerisate kann bei Bedarf auf einen Zusatz von geruchsüberdeckenden Parfümölen verzichtet werden.
Aus den genannten Gründen eignen sich die Acrylatpolymerisate insbesondere als Filmbildner in haarkosmetischen Zubereitungen.

Die Acrylatpolymerisate werden üblicherweise in 0,1 bis 20 Gew.-% vorzugsweise 0,5 bis 10 Gew.-%, insbesondere 2 bis 10 Gew.-% des teilweise oder vollständig neutralisierten Acrylatpolymerisates bezogen auf die kosmetische Zubereitung eingesetzt.

Bevorzugt ist die Verwendung der Acrylatpolymerisate in kosmetischen Zubereitungen, insbesondere in Haarsprayzubereitungen, welche die folgenden Bestandteile enthalten:
- 0,1 bis 20 Gew.-%, vorzugsweise 0,5 bis 10 Gew.-%, insbesondere 2 bis 6 Gew.-% des teilweise oder vollständig neutralisierten Acrylatpolymerisates
- 1 bis 99,9 Gew.-%, vorzugsweise 5 bis 50 Gew.-%, insbesondere 10 bis 20 Gew.-% Wasser
- 0 bis 95 Gew.-%, vorzugsweise 20 bis 60 Gew.-%, insbesondere 25 bis 50 Gew.-% eines üblichen organischen Lösungsmittels wie vor allem Ethanol, Isopropanol und Dimethoxymethan und daneben auch Aceton, n-Propanol, n-Butanol, 2-Methoxypropan-1-ol, n-Pentan, n-Hexan, Cyclohexan, n-Heptan, n-Octan oder Dichlormethan oder deren Gemische
- 0 bis 90 Gew.-%, vorzugsweise 30 bis 80 Gew.-%, insbesondere 45 bis 60 Gew.-% eines üblichen Treibmittels wie n-Propan, iso-Propan, n-Butan, Isobutan, 2,2-Dimethylbutan, n-Pentan, Isopentan, Dimethylether, Difluorethan, Fluortrichlormethan, Dichlordifluormethan oder Dichlortetrafluorethan, HCF 152 A oder deren Gemische

Als Treibmittel (Treibgase) kommen von den genannten Verbindungen vor allem die Kohlenwasserstoffe, insbesondere Propan, n-Butan, n-Pentan und Gemische hieraus sowie Dimethylether und Difluorethan zur Anwendung. Gegebenenfalls werden einer oder mehrere der genannten chlorierten Kohlenwasserstoffe in Treibmittelmischungen mitverwendet, jedoch nur in geringen Mengen, etwa bis zu 20 Gew.-%, bezogen auf die Treibmittelmischung.

Die erfindungsgemäßen haarkosmetischen Zubereitungen eignen sich auch besonders für Pumpsprayzubereitungen ohne den Zusatz von Treibmitteln oder auch für Aerosolsprays mit üblichen Druckgasen wie Stickstoff, Druckluft oder Kohlendioxid als Treibmittel.

Eine wasserhaltige Standard-Sprayformulierung weist beispielsweise die folgende Zusammensetzung auf:
- 2 bis 10 Gew.-%: des zu 100 % mit 2-Amino-2-methylpropanol neutralisierten Acrylatpolymerisates
- 10 bis 76 Gew.-%: Ethanol
- 2 bis 20 Gew.-%: Wasser
- 10 bis 60 Gew.-%: Dimethylether und/oder Propan/ n-Butan und/oder Propan/iso-Butan

Zur gezielten Einstellung der Eigenschaften von haarkosmetischen Zubereitungen kann es von Vorteil sein, die erfindungsgemäßen Acrylatpolymerisate als Mischung mit weiteren Haarfestigungspolymeren einzusetzen.

Als Haarkosmetik-Polymere eignen sich beispielsweise anionische Polymere. Solche anionischen Polymere sind von den erfindungsgemäßen Acrylatpolymerisaten verschiedene Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (z.B. Luviset P.U.R) und Polyharnstoffe. Besonders geeignete Polymere sind, Copolymere aus Ethylacrylat und Methacrylsäure (z.B. Luvimer®MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester(z.B. Luviset®- Marken), Maleinsäureanhydridcopolymere, ggf. mit Alkoholen umgesetzt, anionische Polysiloxane, z.B. carboxyfunktionelle Copolymere aus Vinylpyrrolidon, t-Butylacrylat, Methacrylsäure (z.B. Luviskol® VBM) oder Terpolymere aus tert.Butylacrylat, Methacrylsäure und Dimethicone Copolyol (z.B. Luviflex® Silk).

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Cellulosederivate, Polyasparaginsäuresalze und Derivate.

Die Acrylatpolymerisate können in kosmetischen Zubereitungen mit üblichen weiteren Hilfsstoffen formuliert werden. Als weitere übliche Hilfsstoffe seien genannt: Tenside, Ölkörper, Emulgatoren, Co-Emulgatoren, Überfettungsmittel, Perlglanzwachse, Konsistenzgeber, Verdickungsmittel, Fette, Wachse, Silikonverbindungen, Hydrotrope, Konservierungsmittel, Parfümöle, Farbstoffe, Stabilisatoren, pH-Wert Regulatoren, Pflegestoffen wie Panthenol, Phytantriol, Collagen, Vitamine und Eiweißstoffe, Solubilisatoren, Glittersubstanzen, Komplexbildner und dergleichen enthalten.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate- oder -propionate, Alkylamphodiacetate, oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglykoside oder Sorbitanetherester geeignet.

Außerdem können die Mittel übliche kationische Tenside enthalten, wie z.B. quaternäre Ammoniumverbindungen, beispielsweise Cetyltrimethylammoniumchlorid.

Werden die erfindungsgemäßen Acrylatpolymerisate in Shampooformulierungen eingesetzt, so enthalten diese üblicherweise anionische Tenside als Basistenside und amphotere und nichtionische Tenside als Cotenside.

Die kosmetischen Zubereitungen enthalten üblicherweise 2 bis 50 Gew.-% Tenside, bevorzugt 5 bis 40 Gew.-%, besonders bevorzugt 8 bis 30 Gew-%.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglycose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE-PS 2024051 als Rückfettungsmittel für kosmetische Zubereitungen bekannt. C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidesters gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solcher oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Perlglanzwachse kommen beispielsweise in Frage: Alkylenglycolester, spezielle Ethylenglycoldisterat; Fettsäurealkanolamide, speziell Kokosfettsäurediethanoamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder FettsäureN-methylglucamiden gleicher Kettenlänge und/oder Polyglycerinpoly-12-hydroxystearaten. Geeignete Verdickungsmittel sind beispielsweise Polysaccharide, insbesondere Xanthan-Gum, Guar-Guar, Agar-Agar, Alginate und Tylosen, Carboxymethcellulose und Hydroxyethylcellulose, ferner höhermolekulare Polyethylenglycolmono- und -diester von Fettsäuren, Polyacrylate (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Typische Beispiele für Fette sind Glyceride, als Wachse kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als Stabilisatoren können Metallsalze von Fettsäuren, wie z.B. Magnesium-, Calcium-, Aluminium- und/oder Zinkstearat bzw. -ricinoleat eingesetzt werden.

Geeignete Silikonverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Silikonverbindungen, die bei Raumtemperatur sowohl flüssig als auch harzförmig vorliegen können. Typische Beispiele für Fette sind Glyceride, als Wachse kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als Stabilisatoren können Metallsalze von Fettsäuren, wie z.B. Magnesium-, Calcium-, Aluminium- und/oder Zinkstearat eingesetzt werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1000 Dalton;
   technische Oligoglyceringemische mit einem Eigenkondensationsgrad von 1,5 bis 10 wie etwa technische Diglyceringemische mit einem Diglyceringehalt von 40 bis 50 Gew.-%;
- Metylolverbindungen, wie insbesondere Trimethylolethan, Trimetylolpropan, Trimetylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit;
- Zucker mit 5 bis 12 Kohlenstoffatomen, wie beispielsweise Glucose oder Saccharose;
- Aminozucker, wie beispielsweise Glucamin.

Als Konservierungsmittel eignen sich beispielsweise Phenoxyethanol, Formaldehydlösung, Parabene, Pentandiol oder Sorbinsäure sowie die in Anlage 6, Teil A und B, der Kosmetikverordnung aufgeführten weiteren Stoffklassen.

Der Zusatz von Parfümölen zur Geruchüberdeckung der Polymerisate ist nicht erforderlich. Gegebenenfalls können die kosmetischen Zubereitungen trotzdem Parfümöle enthalten. Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetischen Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonat, zu den Ketonen z.B. die Jonone, cc-Isomethylionen und Methylcedrylketon, zu den Alkoholen Anethof, Citronellol, Eugenol, Isoeugenol, Geraniol, Linalool, Phenylethylalkohol und Terioneol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzeöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, a-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, b-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81-106, zusammengestellt sind. Diese Farbstoffe werden üblicherweise in Konzentrationen von 0,001 bis 0,1 Gew.-%, bezogen auf die gesamte Mischung, eingesetzt.

Weitere geeignete Polymere sind z.B. kationische Polymere mit der Bezeichnung Polyquaternium nach INCI wie z.B. Copolymere aus N-Vinylpyrrolidon/N-Vinylimidazoliumsalzen (Luviquat®FC, Luviquat ®HM, Luviquat® MS, Luviquat Care®), Copolymere aus N-Vinylcaprolactam/N-Vinylpyrrolidon/N-Vinylimidazoliumsalzen (Luviquat Hold®), Copolymere aus N-Vinylpyrrolidon/Dimethylaminoethylmethacrylat, quaternisiert mit Diethylsulfat (Luviquat® PQ11), kationische Cellulosederivate (Polyquaternium-4 und -10), Acrylamidcopolymere (Polyquaternium-7) und Guar-hydroxypropyltrimethylammoniumchlorid (INCI: Hydroxypropyl Guar Hydroxypropyltrimonium Chloride. Geeignete Polymere sind auch Polyethylenimine und deren Salze, Polyvinylamine und deren Salze.

Der Gesamtanteil der Hilfs- und Zusatzstoffe kann 1 bis 50, vorzugsweise 5 bis 40 Gew.-% - bezogen auf die Mittel - betragen.

Ein weiterer Gegenstand der vorliegenden Erfindung betrifft ein Verfahren zur Herstellung von Acrylatpolymerisaten, bei dem man Alkanthiole, insbesondere lineare Alkanthiole mit einer C-Kettenlänge von C 14 bis C 22 einsetzt. Die Alkanthiole werden dabei üblicherweise zusammen mit den polymerisierbaren Monomeren eingesetzt. Die Alkanthiole werden hierbei üblicherweise in Mengen von 0,1 bis 5 Gew.-% bezogen auf die polymerisierbaren Monomere eingesetzt.

Ein weiterer Gegenstand der vorliegenden Erfindung betrifft eine Verfahren zur Herstellung von Acrylatpolymerisaten, bei dem man Alkanthiole, insbesondere lineare Alkanthiole mit einer C-Kettenlänge von C 10 bis C 22 einsetzt und nach Abschluß der Polymerisation eine Wasserstoffperoxid Behandlung durchführt.

Die nach den erfindungsgemäßen Verfahren erhältlichen Acrylatpolymerisate zeichnen sich durch geringen bis keinen Eigengeruch aus.

Die erfindungsgemäßen Verfahren eignen sich allgemein zur Herstellung von Acrylatpolymerisaten, insbesondere denen in Ansprüchen 1 und 2 beschriebenen.

Darüber hinaus eignen sich die erfindungsgemäßen Verfahren zur Herstellung von weiteren Acrylatpolymerisaten.

Als Acrylatpolymerisate gelten alle Polymerisate, die zumindest ein Monomer auf Acrylatbasis enthalten.

Repräsentative aber nicht limitierende Beispiele von geeigneten Monomeren sind zum Beispiel Acrylsäure und deren Salze, Ester und Amide. Die Salze können von jedem beliebigen nicht toxischen Metall, Ammonium oder substituierten Ammonium-gegenionen abgeleitet sein.

Die Ester können abgeleitet sein von C₁-C₄₀ linearen, C₃-C₄₀ verzweigtkettigen, oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin, and 1,2,6-Hexantriol, von Aminoalkoholen oder von Alkoholethern wie Methoxyethanol und Ethoxyethanol oder Polyethylenglykolen.

Ferner eignen sich N,N'-Dialkylaminoalkylacrylate- und methacrylate und N-Dialkylaminoalkylacryl- und -methacrylamide der allgemeinen Formel (I) 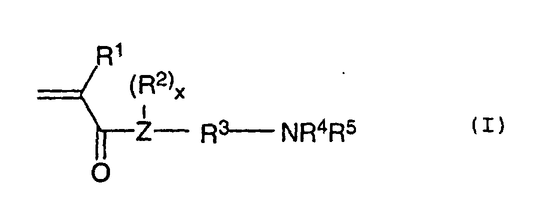 mit
- R¹ =: H, Alkyl mit 1 bis 8 C-Atomen,
- R² =: H, Methyl,
- R³ =: Alkylen mit 1 bis 24 C-Atomen, optional substituiert durch Alkyl,
- R⁴, R⁵ =: C₁-C₄₀ Alkylrest,
- Z =: Stickstoff für x = 1 oder Sauerstoff für x = 0

Die Amide können unsubstituiert, N-Alkyl oder N-alkylamino monosubstituiert, oder N,N-dialkylsubstituiert oder N,N-dialkylamino disubstituiert, worin die Alkyl- oder Alkylaminogruppen von C1-C40 linearen, C3-C40 verzweigtkettigen, oder C3-C40 carbocyclischen Einheiten abgeleitet sind. Zusätzlich können die Alkylaminogruppen quarternisiert werden.

Bevorzugte Monomere der Formel I sind N,N-Dimethylaminomethyl-(meth)acrylat, N,N-Diethylaminomethyl(meth)acrylat, N,N-Dimethylaminoethyl(meth)acrylat, N,N-Diethylaminoethyl(meth)acrylat.

Ebenfalls verwendbare Monomere sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C1-C4 Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Besonders geeignete Monomere sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydrohyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;

Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylimidazol, N,N-Dimethylaminomethyl(meth)acrylat, N,N-Diethylaminomethyl(meth)acrylat, N,N-Dimethylaminoethyl(meth)acrylat, N,N-Diethylaminoethyl(meth)-acrylat, N,N-Dimethylaminobutyl(meth)acrylat, N,N-Diethylaminobutyl(meth)acrylat, N,N-Dimethylaminohexyl(meth)acrylat, N,N-Dimethylaminooctyl(meth)acrylat, N,N-Dimethylaminododecyl (meth)acrylat, N-[3-(dimethylamino)propyl]methacrylamid, N-[3-(dimethylamino)propyl]acrylamid, N-[3-(dimethylamino)butyl]methacrylamid, N-[8-(dimethylamino)octyl]methacrylamid, N-[12-(dimethylamino)dodecyl]methacrylamid, N-[3-(diethylamino)propyl]-methacrylamid, N-[3-(diethylamino)propyl]acrylamid;

Von diesen sind besonders bevorzugt Acrylsäure, Methacrylsäure, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, N-t-Butylacrylamid, N-Octylacrylamid, 2-Hydroxyethylacrylat, Hydroxypropylacrylat, 2-Hydroxyethylmethacrylat, Hydroxypropylmethacrylat, Alkylenglykol(meth)-acrylate, ungesättigte Sulfonsäuren wie zum Beispiel Acrylamidopropansulfonsäure oder Diethylsulfat.

Die erfindungsgemäßen Verfahren eignen sich insbesondere für die Herstellung von Homo- und Copolymerisaten von Acrylsäure und Acrylamid und deren Salze, Copolymeren aus tert.-Butylacrylat, Ethylacrylat, Methacrylsäure (z.B. Luvimer™ 100 P), Copolymere aus Ethylacrylat und Methacrylsäure, (z.B. Luviflex™ Soft), Copolymere aus N-tert.-Butylacrylamid, Ethylacrylat, Acrylsäure (Ultrahold Strong™), Copolymere von Acrylsäure und Methacrylsäure mit hydrophoben Monomeren, z.B. C₄-C₃₀-Alkylester der (Meth)acrylsäure, C₄-C₃₀-Alkylvinylester, C₄-C₃₀-Alkylvinylether und Hyaluronsäure so wie weitere unter den Handelsnamen bekannte Polymere Amerhold DR-25, Ultrahold™, Acronal™, Acudyne™, Lovocryl™, Versatyl™, Amphomer™ (28-4910, LV-71), Placise™ L53, Advantage Plus™, Balance™ (0/55), Acudyne™ 255.

Die nachfolgende Liste enthält die INCI/CTFA-Bezeichnungen sowie die Hersteller, der Acrylatpolymerisate für die die erfindungsgemäßen Verfahren geeignet sind:

| INCI/CTFA | Polymer | Hersteller |
|---|---|---|
| Acrylates Copolymer | Amerhold DR-25 | Amerchol |
| Styrene/Acrylates Copolymer | Acronal 290 D, 296 D | BASF |
| Acrylates/Acrylamide Copolymer | Ultrahold 8 | BASF |
| Acrylates/Acrylamide Copolymer | Ultrahold Strong | BASF |
| Acrylates Copolymer | Luviflex Soft | BASF |
| Acrylates Copolymer | Luvimer 100P, 36D, 30E | BASF |
| Methacryloyl Ethylbetaine/ Acrylates Copolymer | Diaformer | Clariant |
| Acrylates/Diacetoneacrylamide Copolymer | Plascize L-53 | Goo Chemical |
| Vinyl Caprolactam/ PVP/Dimethylazninoethyl Methacrylate Copolymer | Copolymer VC 713 (= Advantage HC) | ISP |
| Vinyl Caprolactam/ PVP/Dimethylaminoethyl Methacrylat Copolymer | H₂OLD® EP-1 | ISP |
| VA/Butyl Maleate/Isobornyl Acrylate | Advantage Plus | ISP |
| PVP/DMAPA Acrylates Copolymer | Styleeze CC-10 | ISP |
| PVP/Vinylcaprolactam/DMAPA Acrylates Copolymer | Aquaflex SF-40 | ISP |
| Octylacrylamide/Acrylates/ Butylaminoethyl Methacrylate Copolymer | Amphomer 28-4910 | National Starch |
| Octylacrylamide/Acrylates/ Butylaminoethyl Methacrylate Copolymer | Amphomer LV-71 | National Starch |
| Acrylates/Octylacrylamide Copolymer | Versatyl 42 | National Starch |
| Octylacrylamide/Acrylates Copolymer | Versatyl 90 | National Starch |
| Acrylates Copolymer | Balance 0/55 | National Starch |
| Octylacrylamide/Acrylates/Butylaminoethyl Methacrylate Copolymer | Lovocryl 47 | National Starch |
| Acrylates/Hydroxyesters Acrylates | Acudyne | Rohm & Haas |

Die Polymerisationsverfahren können durchgeführt werden als Lösungspolymerisation, Emulsionspolymerisation, umgekehrte Emulsionspolymerisation, Suspensionspolymerisation, umgekehrte Suspensionspolymerisation oder Fällungspolymerisation sein, ohne daß die verwendbaren Methoden darauf beschränkt sind. Bei der Lösungspolymerisation können Wasser oder übliche organische Lösungsmittel als Lösungsmittel verwendet werden.

### Beispiele

### Beispiel 1 Herstellung eines Acrylatpolymerisates B 5 (Emulsionspolymerisation)

Aus 2,5 g Natriumlaurylsulfat, 15,6 g eines handelsüblichen nichtionischen Emulgators, 300 g Wasser, 140 g Methacrylsäure (Monomer B), 490 g tert.-Butylacrylat (Monomer A), 70 g Ethylacrylat (Monomer C) und 3 g tert.-Dodecylmercaptan, wurde eine Emulsion hergestellt. Diese Emulsion wurde im Zulaufverfahren in ein Polymerisationsgefäß, das 750 g Wasser enthielt, innerhalb einer Polymerisationsdauer von ca. 2 bis 4 Stunden bei ca. 75 bis 85°C zudosiert. Der Polymerisationsinitiator, 1,1 g Natriumpersulfat gelöst in 14,9 g Wasser, wurde bei Zulaufbeginn der Emulsion zugegeben. Nach Ende der Polymerisation wurden bei 60 bis 70°C 3,6 g Wasserstoffperoxid (50 %ig) zudosiert.

Die Herstellung der übrigen Beispiele (B1 bis B4 und B6 bis B 9) sowie der Vergleichsbeispiele V1 (gemäß EP 696 916) bis V 5 erfolgte entsprechend Beispiel 1. Die entsprechenden Mengen an Monomeren A, B und C sowie die Art und Menge an Alkanthiol sowie gegebenenfalls Wasserstoffperoxid sind der folgenden Tabelle zu entnehmen.

Die geruchliche Beurteilung der hergestellten Acrylatpolymerisate erfolgte durch ein Panel von 4 Prüfern. Dazu wurden die gemäß den Beispiele hergestellten Acrylatpolymerisate getrocknet, 30 % in Ethanol gelöst und anschließend als 3 %ige wässerige Lösung formuliert und zu 100 % mit 2-Amino-2-methylpropanol neutralisiert. Die Bewertung der geruchlichen Eigenschaften erfolgt nach folgender Klassifizierung:
"1" kein Geruch
"2" schwacher, unangenehmer Geruch
"3" starker, unangenehmer Geruch

| Beisp Nr. | Zusammensetzung [Gew.-%] | | | | Zusatz | Zusatz [Gew.-%] | H₂O₂-Menge [Gew.-%] | Geruch |
|---|---|---|---|---|---|---|---|---|
| | t-BA | MAS | EA | t-BAA | | | | |
| V 1 | 70 | 18 | 12 | - | Mercaptoethanol | 0,3 | - | 2 |
| V 2 | 72 | 18 | 10 | - | n-Butanthiol | 0,15 | - | 3 |
| V 3 | 72 | 18 | 10 | - | n-Butanthiol | 0,15 | 0,40 | 3 |
| V 4 | 65 | 15 | 20 | - | 1-Octanthiol | 0,45 | 0,50 | 3 |
| | | | | | | | | |
| B 1 | 65 | 15 | 20 | - | Decanthiol | 0,50 | 0,30 | 1 |
| B 2 | 65 | 20 | 5 | 10 | Decanthiol | 0,50 | 0,40 | 1 |
| B 3 | 65 | 15 | 20 | - | Decanthiol | 0,50 | 0,50 | 1 |
| | | | | | | | | |
| V 5 | 65 | 15 | 10 | 10 | n-Dodecanthiol | 0,45 | - | 2 |
| V 6 | 70 | 20 | 10 | - | tert-Dodecanthiol | 0,40 | - | 2 |
| | | | | | | | | |
| B 4 | 65 | 15 | 10 | 10 | n-Dodecanthiol | 0,45 | 0,30 | 1 |
| B 5 | 70 | 20 | 10 | - | n-Dodecanthiol | 0,40 | 0,25 | 1 |
| B 6 | 67 | 23 | 10 | - | n-Dodecanthiol | 0,43 | 0,50 | 1 |
| B 7 | 67 | 23 | 10 | - | n-Dodecanthiol | 0,4 | 0,11 | 1 |
| B 8 | 50 | 20 | 20 | 10 | n-Dodecanthiol | 0,35 | 0,40 | 1 |
| | | | | | | | | |
| B 9 | 67 | 23 | 10 | - | n-Octadecanthiol | 0,50 | 0,20 | 1 |
| B 10 | 60 | 20 | 20 | - | n-Octadecanthiol | 0,50 | 0,50 | 1 |
| | | | | | | | | |

Erklärungen zur Tabelle
t-BA = tert.-Butylacrylat
MAS = Methacrylsäure
AS = Acrylsäure
EA = Ethylacrylat
t-BAA = N-tert.-Butylacrylamid

| Rezepturbeispiele | | | | | |
|---|---|---|---|---|---|
| Inhaltsstoff | INCI | Rezeptur 1 Aerosol Haarspray | Rezeptur 2 Aerosol Haarspray | Rezeptur 3 Aerosol Haarspray | Rezeptur 4 Pumpspray |
| | | | Angaben in Gew.-% | | |
| Acrylatpolymerisat gemäß Beispiel 1, zu 100 % neutralisiert mit AMP | Acrylate Copolymer | 4,0 | 2,0 | 4,0 | 5,0 |
| 2-Amino-2-methylpropanol (AMP) | Aminomethyl Propanol | 0,95 | 0,47 | 0,93 | 1,18 |
| Wasser | Water | - | - | 7,15 | 13,82 |
| Dow Corning 190 Polyether oder Wacker DMC 6031 | Dimethicone Copolyole | 0,1 | | 0,01 | |
| Luvitol EHO | Cetearyl Octanoate | 0,1 | | | |
| Dow Corning 344 fluid oder Wacker CM 040 | Cyclomethicone | | | 0,05 | |
| D-Panthenol USP | Panthenol | | 0,1 | | |
| Abil B 8843 oder Wacker DMC 6032 | Dimethicone Copolyole | | 0,1 | | |
| Ethanol abs. | Alcohol | 54,75 | 47,23 | | 80,00 |
| Ethanol 96 % | Alcohol | | | 42,77 | |
| Propane/Butane | Propane/Butane | 40,0 | 50,0 | | |
| Dimethyl Ether | Dimethyl Ether | | | 45,0 | |

## Patentansprüche

1. Acrylatpolymerisate mit einem K-Wert von 10 bis 60 erhältlich durch radikalische Polymerisation von
- 30 bis 99 Gew.-% tert.-Butylacrylat und/oder tert.-Butylmethacrylat als Monomerem A,
- 1 bis 28 Gew.-% Acrylsäure und/oder Methacrylsäure als Monomerem B und
- 0 bis 60 Gew.-% eines radikalisch copolymerisierbaren Monomeren oder einer radikalisch copolymerisierbaren Monomerenmischung als Monomerem C, wobei mindestens eines der Monomeren C ein Homopolymerisat mit einer Glastemperatur kleiner als 30°C liefert,
mit der Maßgabe, daß sich die Gew.-% zu 100 addieren, in Gegenwart von Alkanthiolen mit der C-Kettenlänge von C 14 bis C 22.

2. Acrylatpolymerisate mit einem K-Wert von 10 bis 60, erhältlich durch radikalische Polymerisation von
- 30 bis 99 Gew.-% tert.-Butylacrylat und/oder tert.-Butylmethacrylat als Monomerem A,
- 1 bis 28 Gew.-% Acrylsäure und/oder Methacrylsäure als Monomerem B und
- 0 bis 60 Gew.-% eines radikalisch copolymerisierbaren Monomeren oder einer radikalisch copolymerisierbaren Monomerenmischung als Monomerem C, wobei mindestens eines der Monomeren C ein Homopolymerisat mit einer Glastemperatur kleiner als 30°C liefert,
mit der Maßgabe, daß sich die Gew.-% zu 100 addieren
in Gegenwart von Alkanthiolen mit der C-Kettenlänge von C 10 bis C 22
und anschließender Wasserstoffperoxid-Behandlung,

3. Acrylatpolymerisate nach den Ansprüchen 1 und/oder 2, **dadurch gekennzeichnet, daß** man lineare Alkanthiole einsetzt.

4. Acrylatpolymerisate nach den Ansprüchen 1 und/oder 2, **dadurch gekennzeichnet, daß** man 0,1 bis 5 Gew.-% Alkanthiole - bezogen auf die polymerisierbaren Monomere - einsetzt.

5. Acrylatpolymerisate nach den Ansprüchen 1 und/oder 2, **dadurch gekennzeichnet, daß** das Monomere C ausgewählt ist aus der Gruppe, die gebildet wird von C₁- bis C₁₈-Alkylacrylaten, C₁- bis C₁₈- Methacrylaten, N-C₁- bis -C₁₈-Alkylacrylamiden und N-C₁- bis -C₁₈-Methacrylamiden.

6. Acrylatpolymerisate nach einem der zuvor genannten Ansprüche, **dadurch gekennzeichnet, daß** man
A) 30 bis 72 Gew.-% des Monomeren A,
B) 10 bis 28 Gew.-% des Monomeren B und
C) 0 bis 60 Gew.-% des Monomeren C
einsetzt mit der Maßgabe, daß sich die Gew.-% zu 100 addieren.

7. Acrylatpolymerisat nach einem der zuvor genannten Ansprüche, **dadurch gekennzeichnet, daß** man
A) tert-Butylacrylat als Monomeres A
B) Methacrylsäure als Monomeres B
C) Ethylacrylat oder eine Mischung aus Ethylacrylat und N-tert.-Butylacrylamid als Monomeres C
einsetzt.

8. Verwendung eines Acrylatpolymerisates nach einem der zuvor genannten Ansprüche als Filmbildner.

9. Verwendung eines Acrylatpolymerisates nach einem der zuvor genannten Ansprüche in kosmetischen Zubereitungen.

10. Verwendung nach Anspruch 9 in haarkosmetischen Zubereitungen.

11. Verfahren zur Herstellung von Acrylatpolymerisaten, **dadurch gekennzeichnet, daß** man die Polymerisation in Gegenwart von Alkanthiolen mit einer C-Kettenlänge von C 14 bis C 22 durchführt.

12. Verfahren zur Herstellung von Acrylatpolymerisaten, **dadurch gekennzeichnet, daß** man
a) die Polymerisation in Gegenwart von Alkanthiolen mit einer C-Kettenlänge von C 10 bis C 22 durchführt und anschließend
b) eine Behandlung mit Wasserstoffperoxid durchführt.

13. Verfahren nach den Ansprüchen 11 und/oder 12, **dadurch gekennzeichnet, daß** man 0,1 bis 5 Gew.-% Alkanthiol - bezogen auf die Menge an zu polymerisierenden Monomeren - einsetzt.

14. Verfahren nach Anspruch 12, **dadurch gekennzeichnet, daß** man die Behandlung mit 0,01 bis 2 Gew.-% Wasserstoffperoxid - bezogen auf die Menge an zu polymerisierenden Monomeren - durchführt.

15. Verwendung von Alkanthiolen mit einer C-Kettenlänge von C 14 bis C 22 zur Herstellung von Acrylatpolymerisaten.

## Claims

1. An acrylate polymer with a K value of from 10 to 60 obtainable by free-radical polymerization of
- 30 to 99% by weight of tert-butyl acrylate and/or tert-butyl methacrylate as monomer A,
- 1 to 28% by weight of acrylic acid and/or methacrylic acid as monomer B and
- 0 to 60% by weight of a free-radically copolymerizable monomer or a free-radically copolymerizable monomer mixture as monomer C, where at least one of the monomers C produces a homopolymer with a glass temperature of less than 30°C,
with the proviso that the % by weight add up to 100,
in the presence of alkanethiols with a carbon chain length of from C 14 to C 22.

2. An acrylate polymer with a K value of from 10 to 60, obtainable by free-radical polymerization of
- 30 to 99% by weight of tert-butyl acrylate and/or tert-butyl methacrylate as monomer A,
- 1 to 28% by weight of acrylic acid and/or methacrylic acid as monomer B and
- 0 to 60% by weight of a free-radically copolymerizable monomer or a free-radically copolymerizable monomer mixture as monomer C, where at least one of the monomers C produces a homopolymer with a glass transition temperature of less than 30°C,
with the proviso that the % by weight add up to 100
in the presence of alkanethiols with a carbon chain length of from C 10 to C 22
and subsequent hydrogen peroxide treatment.

3. An acrylate polymer as claimed in claims 1 and/or 2, wherein linear alkanethiols are used.

4. An acrylate polymer as claimed in claims 1 and/or 2, wherein 0.1 to 5% by weight of alkanethiols, based on the monomers to be polymerized, are used.

5. An acrylate polymer as claimed in claims 1 and/or 2, wherein the monomer C is chosen from the group formed by C₁₋ to C₁₈-alkyl acrylate, C₁₋ to C₁₈-alkyl methacrylate, N-C₁- to -C₁₈-alkylacrylamides and N-C₁- to -C₁₈-alkylmethacrylamides.

6. An acrylate polymer as claimed in any of the abovementioned claims, wherein
A) 30 to 72% by weight of the monomer A,
B) 10 to 28% by weight of the monomer B and
C) 0 to 60% by weight of the monomer C
are used, with the proviso that the % by weight add up to 100.

7. An acrylate polymer as claimed in any of the abovementioned claims, wherein
A) tert-butyl acrylate is used as monomer A
B) methacrylic acid is used as monomer B
C) ethyl acrylate or a mixture of ethyl acrylate and N-tert-butylacrylamide is used as monomer C.

8. The use of an acrylate polymer as claimed in any of the abovementioned claims as film former.

9. The use of an acrylate polymer as claimed in any of the abovementioned claims in cosmetic preparations.

10. The use as claimed in claim 9 in hair cosmetic preparations.

11. A process for the preparation of acrylate polymers, which comprises carrying out the polymerization in the presence of alkanethiols with a carbon chain length of from C 14 to C 22.

12. A process for the preparation of acrylate polymers, which comprises
a) carrying out the polymerization in the presence of alkanethiols with a carbon chain length of from C 10 to C 22 and then
b) carrying out a treatment with hydrogen peroxide.

13. A process as claimed in claims 11 and/or 12, wherein 0.1 to 5% by weight of alkanethiol, based on the amount of monomers to be polymerized, is used.

14. A process as claimed in claim 12, wherein the treatment is carried out with 0.01 to 2% by weight of hydrogen peroxide, based on the amount of monomers to be polymerized.

15. The use of alkanethiols with a carbon chain length of from C 14 to C 22 for the preparation of acrylate polymers.

## Revendications

1. Polymères acryliques présentant une valeur K de 10 à 60, que l'on peut obtenir par polymérisation radicalaire
- de 30 à 99% en poids d'acrylate de butyle tertiaire et/ou de méthacrylate de butyle tertiaire, comme monomère A,
- de 1 à 28% en poids d'acide acrylique et/ou d'acide méthacrylique, comme monomère B, et
- de 0 à 60% en poids d'un monomère copolymérisable par voie radicalaire ou d'un mélange de monomères copolymérisable par voie radicalaire, comme monomère C, au moins un des monomères C fournissant un homopolymère ayant une température de transition vitreuse inférieure à 30°C,
à la condition que les % en poids s'additionnent pour donner 100,
en présence d'alcanethiols ayant une longueur de chaîne de 14 C à 22 C.

2. Polymères acryliques présentant une valeur K de 10 à 60, que l'on peut obtenir par polymérisation radicalaire
- de 30 à 99% en poids d'acrylate de butyle tertiaire et/ou de méthacrylate de butyle tertiaire, comme monomère A,
- de 1 à 28% en poids d'acide acrylique et/ou d'acide méthacrylique, comme monomère B, et
- de 0 à 60% en poids d'un monomère copolymérisable par voie radicalaire ou d'un mélange de monomères copolymérisable par voie radicalaire, comme monomère C, au moins un des monomères C fournissant un homopolymère ayant une température de transition vitreuse inférieure à 30°C,
à la condition que les % en poids s'additionnent pour donner 100,
en présence d'alcanethiols ayant une longueur de chaîne de 10 C à 22 C,
et par traitement ultérieur au peroxyde d'hydrogène.

3. Polymères acryliques suivant les revendications 1 et/ou 2, **caractérisés en ce qu'**on met en oeuvre des alcanethiols linéaires.

4. Polymères acryliques suivant les revendications 1 et/ou 2, **caractérisés en ce qu'**on met en oeuvre 0,1 à 5% en poids d'alcanethiols, par rapport aux monomères polymérisables.

5. Polymères acryliques suivant les revendications 1 et/ou 2, **caractérisés en ce que** le monomère C est choisi parmi le groupe formé des acrylates d'alkyle en C₁-C₁₈, des méthacrylates d'alkyle en C₁-C₁₈, des N-C₁-C₁₈-alkylacrylamides et des N-C₁-C₁₈-alkylméthacrylamides.

6. Polymères acryliques suivant l'une des revendications précédentes, **caractérisés en ce qu'**on met en oeuvre
A) 30 à 72% en poids du monomère A,
B) 10 à 28% en poids du monomère B, et
C) 0 à 60% en poids du monomère C,
à la condition que les % en poids s'additionnent pour donner 100.

7. Polymère acrylique suivant l'une des revendications précédentes, **caractérisé en ce qu'**on met en oeuvre
A) de l'acrylate de butyle tertiaire, comme monomère A,
B) de l'acide méthacrylique, comme monomère B,
C) de l'acrylate d'éthyle ou un mélange d'acrylate d'éthyle et de N-tert-butylacrylamide, comme monomère C.

8. Utilisation d'un polymère acrylique suivant l'une des revendications précédentes, comme agent filmogène.

9. Utilisation d'un polymère acrylique suivant l'une des revendications précédentes, dans des compositions cosmétiques.

10. Utilisation suivant la revendication 9, dans des compositions cosmétiques pour les cheveux.

11. Procédé de préparation de polymères acryliques, **caractérisé en ce qu'**on effectue la polymérisation en présence d'alcanethiols ayant une longueur de chaîne de 14 C à 22 C.

12. Procédé de préparation de polymères acryliques,
**caractérisé en ce que**
a) on effectue la polymérisation en présence d'alcanethiols présentant une longueur de chaîne de 10 C à 22 C, et ensuite
b) on effectue un traitement au peroxyde d'hydrogène.

13. Procédé suivant les revendications 11 et/ou 12, **caractérisé en ce qu'**on met en oeuvre 0,1 à 5% en poids d'alcanethiol par rapport à la quantité de monomères à polymériser.

14. Procédé suivant la revendication 12, **caractérisé en ce qu'**on effectue le traitement avec 0,01 à 2% en poids de peroxyde d'hydrogène, par rapport à la quantité de monomères à polymériser.

15. Utilisation d'alcanethiols présentant une longueur de chaîne de 14 C à 22 C pour la préparation de polymères acryliques.
